# EUROPÄISCHE PATENTSCHRIFT

(11) **EP 2 505 221 B1**
(45) Veröffentlichungstag und Bekanntmachung des Hinweises auf die Patenterteilung: **18.12.2013**
(21) Anmeldenummer: 12174064.1
(22) Anmeldetag: 24.09.2010
(51) Int. Cl.: A61M 16/16

(54) **Vorrichtung zur Befeuchtung von Atemluft für die künstliche Beatmung**
Device for moistening brethed air for artificial respiration
Dispositif d'humidification d'air inhalé pour la respiration artificielle

(30) Priorität: 06.11.2009 EP 09013976
(43) Veröffentlichungstag der Anmeldung: 03.10.2012
(62) Teilanmeldung aus: 10010536.0
(73) Patentinhaber: Arta Plast AB, 135 48 Tyresö (SE)
(72) Erfinder: Steg, Helge, 135 48 Tyresö (SE); Hall, Leif, 145 60 Norsborg (SE)
(74) Vertreter: Nilsson, Lars

(56) Entgegenhaltungen:
- US-A- 4 192 836
- US-A- 4 366 105
- US-A- 5 195 515
- US-A1- 2004 050 386
- US-A1- 2008 054 500

## Beschreibung

Die Erfindung betrifft eine Vorrichtung zur Befeuchtung von Atemluft für die künstliche Beatmung von Humeniden, insbesondere des Menschen, umfassend wenigstens einen im wesentlichen geschlossenen Behälter zur Aufnahme von Wasser, wobei der Behälter eine erste Öffnung zum Einlaß der unbefeuchteten Luft aufweist sowie eine zweite Öffnung zum Auslaß der befeuchteten Luft aufweist und einen Einlaß für die Zufuhr des Wassers in den Behälter.

Eine solche Vorrichtung ist aus der US 4 366 105 bekannt.

Vorrichtungen dieser Art, die in der medizinischen Terminologie schlicht Luftbefeuchter und in der englischsprachigen Welt "humidifier" genannt werden, werden im ambulanten und im klinischen Bereich gleichermaßen eingesetzt. Sie dienen, wie es der gattungsgemäße Begriff auch schon zutreffend sagt, derBefeuchtung von Atemluft, wenn Menschen aber auch Tiere für die verschiedensten Anlässe bzw. Zwecke künstlich beatmetwerden müssen bzw. zu der normalen Atmung zusätzliche Luft appliziert werden muß. Es ist bekannt, daß die zu einer künstlichen Beatmung bereitgestellte Luft entweder aus unter Druck befindlichen Lufttlaschen oder unmittelbar über Pumpeinrichtungen bereitgestellte Luft regelmäßig nicht den Feuchtigkeitsgrad aufweist, derfür eine schonende künstliche Beatmung des Menschen oder auch eines Tieres notwendig ist. Für diesen Zweck sind im Stand der Technik eine Vielzahl von Vorrichtungen der eingangs genannten Art (humidifier) bekannt, die alle mehr oder weniger die oben eingangs genannte gattungsmäßige Grundkonstruktion aufweisen. Da der Fachwelt der Grundaufbau dieser Vorrichtungen, die Anwendungsbereiche und die speziellen Applizierungen der befeuchteten Atemluft beim Menschen und beim Tier gut bekannt sind, ist ein weiteres Eingehen darauf nicht erforderlich.

Die bekannten Vorrichtungen haben aber alle, soweit erkennbar, den einen und/oder den anderen Nachteil, worauf hier nachfolgend gesondert hinzuweisen ist. Es versteht sich von selbst, daß diese Vorrichtungen bei ihrem Betrieb, d.h. bei ihrer Anwendung zur Befeuchtung der Atemluft, keine Mängel bzw. Störungen in ihrer Funktion zeigen dürfen, wobei ein Hauptproblem, das durch viele unterschiedliche Konstruktionen gelöst werden sollte, das ist, daß auf alle Fälle bei der die Vorrichtung verlassenden befeuchteten Atemluft aus der Vorrichtung stammendes Wasser, das in dieser zur Befeuchtung der Luft verwendet wird, über die Atemluftverbindung zwischen der Vorrichtung und dem Menschen oder dem Tier nicht in deren Lunge gerät. Das kann, ohne darauf im einzelnen noch weiter eingehen zu müssen, fatale Folgen für den zu beatmenden Menschen oder das zu beatmende Tier bis hin zum Tode haben.

Um das zu verhindern, sind in dem Behälter, den die Vorrichtung umfaßt, Ventilkonstruktionen diverser unterschiedlicher Art vorgeschlagen worden, die verhindern sollen, daß Wasser, das bestimmungsgemäß zur Befeuchtung der Atemluft im Behälter aufgenommen wird, aus dem Auslaß mit der befeuchteten Atemluft aus dem Behälter zum zu beatmenden Menschen oder Tier gelangen kann. Die bisher dazu vorgeschlagenen Lösungen, die dieses Problem bzw. diesen unter keinen Umständen zu akzeptierenden Nachteil aufgrund der damit verbundenen gesundheitlichen Schäden lösen sollen, d.h. zur Lösung vorgeschlagen worden sind, basieren alle im wesentlichen auf dem Prinzip, daß zwarimmer nur eine vorbestimmte Menge Wassers im Behälter aufgenommen und nachgefüllt wird, da sich das Wasser im Behälter durch Eintritt in die durch den Behälter durchgeführte Luft verbraucht. Es wird aber nicht verhindert, daß bei Störungen dieservorrichtungseigenen Mittel, die den Nachlauf des Wassers in den Behältersteuern, nicht ausgeschlossen werden kann, daß bei deren Störung Wasser über den Auslaß für die befeuchtete Luft zusätzlich zu dem zu beatmenden Menschen bzw. dem Tier gelangen kann, mit den nachteiligen, oben beispielhaft skizzierten vielfach fatalen Folgen.

Es ist somit Aufgabe der vorliegenden Erfindung, eine Vorrichtung der eingangs genannten Art zu schaffen, mit der mit einfachen Mitteln bei einfachem Aufbau einefortlaufend sichere Funktion derVorrichtung geschaffen werden kann, mit der auch bei Störsituationen, bspw. der ungesteuerten Zufuhr von Wasser in den Behälter, und auf alle Fälle verhindert wird, daß Wasser selbst über den Auslaß für die befeuchtete Luft nach außen bzw. zu dem künstlich zu beatmenden Menschen bzw. dem Tier gelangen kann.

Gelöst wird die Aufgabe gemäß der Erfindung nach Anspruch 1 dadurch, daß der Einlaß im Behälteroberteil ausgebildet ist, an den sich ein den Behälterinnenraum im wesentlichen durchquerendes Rohr anschließt, wobei am freien Ende des Rohres ein Ventil angeordnet ist, das den Pegelstand des über das Rohr zugeführten Wassers derart begrenzt, daß der Behälterboden im Behälterinnenraum im wesentlichen mit Wasser bedeckt ist.

Dieses äußerst vorteilhafte Prinzip erlaubt eine Konstruktion der Vorrichtung im Inneren ihres Behälters derart, daß Wasser im inneren Behälter beim Erreichen des besagten vorbestimmten Pegelstandes keinen Zugang zu dem Behälterteil zwischen dem Einlaß für die unbefeuchtete und dem Auslaß für die befeuchtete Luft in einem Störungsfall hat. Der Pegelstand des über das Rohr zugeführten Wassers ist über die vorgeschlagene konstruktive Maßnahme derart begrenzbar, daß lediglich der Behälterboden im Behälterinnenraum mit Wasser bedeckt ist, bspw. einige Millimeter hoch, bezogen auf den Behälterboden.

Die erfindungsgemäße Lösung hat sich als in hohem Maße sicher im Lichte der gestellten Aufgabe herausgestellt und erfüllt auch die gesetzlich an solche Vorrichtungen gestellten Anforderungen bzw. Parameter.

Gem. einer vorteilhaften Ausgestaltung der Befeuchtungsvorrichtung weist das Ventil einen im wesentlichen zylinderförmigen Abschnitt auf, mit dem es im freien Ende des Rohres aufgenommen wird, und einen im wesentlichen tellerförmigen Abschnitte, mit dem es die Öffnung des freien Endes des Rohres in einer Grundstellung verschließt. Für die Zufuhr des Wassers in den Behälterinnenraum wird der tellerförmige Abschnitt des Ventils durch das durch das Rohr zufließende Wasser bei dem Befüllungsvorgang mit Wasser infolge des geringfügig höheren Drucks des Wassers als dem im Behälterinnenraum herrschenden Druck geringfügig verformt, so daß soviel Wasser eindringen kann, wie es der Innendruck im Behälterinnenraum normalerweise zuläßt.

Derzylinderförmige Abschnitt des Ventils weist vorzugsweise eine Mehrzahl axial verlaufender Rinnen auf, durch die das durch das Rohr zugeführte Wasser über den tellerförmigen Abschnitt, wie vorangehend beschrieben, fließen kann und in den Behälterinnenraum bis zur erfindungsgemäß angestrebten Höhe hineinfließt.

Das Ventil ist schließlich vorzugsweise aus elastomerem Werkstoff ausgebildet, bspw. aus Silikongummi, was im medizinischen Bereich unbedenklich eingesetzt werden kann und den Vorteil hat, daß es hochelastisch eingestellt werden kann, so daß insbesondere der tellerförmige Abschnitt seine bestimmungsgemäße Öffnungs- und Schließfunktion über lange Zeiten störungsfrei ausführen kann und dennoch einen sicheren Sitz mit seinem zylinderförmigen Abschnitt im Rohr gewährleistet.

Gem. einer weiteren anderen vorteilhaften Ausführungsform der Befeuchtungsvorrichtung ist ein Mittel vorgesehen, das in Abhängigkeit des Pegelstandes des Wassers im Behälterinnenraum nach Arteines Bypasses zwischen dem Einlaß der unbefeuchteten Luft und dem Auslaß der befeuchteten Luft außer Kontakt mit dem Wasserdampf oberhalb der Oberfläche des Wassers kurzschließt und bei Unterschreiten des Pegelstandes den Teilinnenraum wieder in Kontakt mit dem Wasser bringt. Der in diesem Zusammenhang genannte unterschrittene Pegelstand ist nicht zwingend dem Pegelstand, gleichzusetzen, der bei der erfindungsgemäßen Lösung das über das Rohr zugeführte Wasser im Behälterinnenraum begrenzt. Allerdings kann es durchaus möglich sein, daß diese beiden Pegelstände gleichhoch bemessen sind bei einer bestimmten noch anderen Ausführungsform der Erfindung. Bei dieservorteilhaften Ausführungsform der Befeuchtungsvorrichtung findet in diesem Fall quasi ein Kurzschluß zwischen dem Einlaß der Luft in den Behälter und dem Auslaß der Luft aus dem Behälter statt, wobei dann lediglich die Restfeuchte in diesem dann kurzgeschlossenen Bereich des Behälters als Befeuchtungsmittel dient, abererfindungsgemäßverhindert wird, daß in diesem Zustand überhaupt Wasser aus dem Auslaß für die befeuchtete Luft aus der Vorrichtung austreten kann. Eine kurzzeitige Applikation des Patienten in diesem Falle mit unbefeuchteter oder nur mit einer Restfeuchte versehenen Atemluft ist regelmäßig unschädlich und erfolgt zudem nur so lange, bis der Pegel des Wassers unterhalb des besagten vorbestimmten Pegels gesunken ist bzw. durch äußere Einflußname abgesenkt wird.

Gem. einer vorteilhaften Ausgestaltung der Befeuchtungsvorrichtung umfaßt das Mittel eine Schwimmeinrichtung, die im Behälterinnenraum im dortigen Wasser schwimmend beim Erreichen des vorbestimmten Pegelstandes gegen ein mit dem Behälter verbundenes erstes Dichtelement stößt und dadurch die Zufuhr von Wasser in den Behälter unterbricht. Der Vorteil dieser Ausgestaltung des Mittels ist, daß keinerlei Hebelteile und Gelenke, die in dem Milieu des Wassers zu Funktionsstörungen neigen, vorhanden sind, so daß auch durch diese Maßnahme, wie eingangs gefordert, neben der guten und sicheren Funktionalität eine einfache und somit auch kostengünstige Ausgestaltung der Befeuchtungsvorrichtung möglich ist, da diese auch in vielen Fällen nur einmal gebraucht und dann entsorgt werden muß, um die Übertragung von Keimen zu vermeiden.

Vorteilhaft ist es dabei, daß die Schwimmeinrichtung der Befeuchtungsvorrichtung ein kragenartiges Element aufweist, das gegen das mit dem Behälter verbundene erste Dichtelement stößt, wenn der vorbestimmte maximale Pegelstand des Wassers im Behälter erreicht ist. Dadurch wird vorteilhafterweise, d.h. im an das zweite Dichtelement angestoßenen Zustand des kragenartigen Elementes, der Einlaß der Luft in den Behälter und der Auslaß der Luft aus dem Behälter über einen verbleibenden Teilinnenraum im wesentlichen eingeschlossen, d.h. es wird quasi ein Bypass, siehe oben, für die Luft vom Einlaß der Luft in den Behälter bis zum Auslaß der Luft aus dem Behälter gebildet, wobei in diesen Bypassraum bzw. verbleibendenTeilinnenraum kein Wasser eindringen kann und, wie oben ebenfalls schon allgemein dargestellt, die zu applizierende, befeuchtete Luft zwar nur noch von der Restfeuchte im Teilinnenraum partizipieren kann, auf alle Fälle aber kein Wasser über den Auslaß der Luft aus dem Behälter zur Beatmung des Menschen oder des Tieres gelangen kann. Auch diese Konstruktion istfrei von beweglichen Teilen und ist damit funktionssicher und ebenfalls kostengünstig bereitstellbar.

Gem. einer weiteren anderen vorteilhaften Ausgestaltung der Befeuchtungsvorrichtung erstreckt sich das für die Zufuhr von Wasser in den Behälter im wesentlichen zentrales Rohr im wesentlichen bis in den Bodenbereich des Behälters, wobei bei dieser Ausgestaltung vorzugsweise die Schwimmeinrichtung einen im wesentlichen zentralen Durchtritt, der wie ein zentrales Innenrohr ausgebildet sein kann, aufweist, in dem das zentrale Rohr verläuft. Hier hat das zentrale Rohr quasi auch eine Führungsfunktion für die Schwimmeinrichtung und eine Leitfunktion für das in den Behälterinnenraum von außen einzuführende Wasser und auch hier gilt wiederum der Vorteil, daß keine Gelenke, Scharniere und/oder Hebel u.dgl. für einen Wasserstopp, wie bei den im Stand der Technik bekannten Vorrichtungen, vorhanden sind, wodurch einerseits eine hohe Funktionsfähigkeit und andererseits eine kostengünstige Bereitstellbarkeit gewährleistet sind.

Vorzugsweise ist die Schwimmeinrichtung als im wesentlichen geschlossener, schwimmfähiger Körper ausgebildet, der somit vorteilhafterweise keinerlei Angriffspunkte für ein Verhaken innerhalb des Behälterinnenraums infolge seiner Aufwärts- und Abwärtsbewegung in Abhängigkeit des Pegelstandes des Wassers im Behälterinnenraum bietet, bezogen auf eine im wesentlichen horizontale Ausrichtung des Behälterbodens als Bezugebene. Prinzipiell kann dieserSchwimmkörperdabei auch als hohles Elementausgestaltetsein, es können aber auch dafür Werkstoffe verwendet werden, siehe auch noch weiter unten, die ein spezifisches Gewichtvon < 1 aufweist, so daß dieser leichter als Wasser und somit im Wasser schwimmfähig ist.

Das oben erwähnte kragenartige Element ist vorteilhafterweise als von der Schwimmeinrichtung gesondertes Teil ausgebildet und an der Schwimmeinrichtung befestigt, wobei diese Konstruktion die Ausbildung der Schwimmeinrichtung einerseits und des kragenartigen Elements andererseits aus unterschiedlichen Werkstoffen ermöglicht, wenn bspw. der eigentliche Schwimmkörper selbst keine elastomeren Eigenschaften haben muß, das kragenartige Element aber, da es eine dichtende Funktion bzw. Aufgabe hat, bspw. aus einem elastomeren Werkstoff bestehen soll. Es sei aber klargestellt, daß es natürlich möglich ist, sowohl das kragenartige Element als auch die Schwimmeinrichtung aus gleichem Werkstoff und/oder einstückig auszubilden.

Das zweite Dichtelement, das bei einer noch anderen vorteilhaften Ausgestaltung der Befeuchtungsvorrichtung vorgesehen ist, ist im wesentlichen zentral am Behälteroberteil angeordnet und ist im wesentlichen im Querschnitt kreisförmig ausgebildet und weist zudem vorteilhafterweise eine im wesentlichen umlaufende Dichtlippe auf. Auch hier gilt, daß das Dichtelement faktisch einstückig mit dem Behälteroberteil ausgebildet sein kann und währenddes Formungsprozesses des Behälteroberteils ausgebildet werden kann, es kann aber auch als gesondertes Teil hergestellt werden und im Zuge der Fertigung der Gesamtvorrichtung im Behälteroberteil befestigt werden, bspw. mittels Klebung, Schweißung oder auch in Form eines Anspritzvorganges.

Eine umlaufende Dichtlippe des Dichtelementes selbst kann vorzugsweise einstückig mit dem Dichtelement ausgebildet sein und dabei auch in bezug auf ihre Elastizität den im einzelnen geforderten mechanischen Bedingungen angepaßt sein, d.h. der Grad der Elastizität der Dichtlippe kann unterschiedlich gegenüber dem Grad des Dichtelementes im übrigen sein.

Die Dichtlippe liegt bei einer noch anderen, sehr vorteilhaften Ausgestaltung der Beschichtungsvorrichtung im Zustand der Unterbrechung der Zufuhr von Wasser in den Behälterinnenraum dichtend auf der Schwimmeinrichtung auf, und zwar frei von zusätzlichen weiteren Dichtmitteln auf der Schwimmeinrichtung, d.h. für diesen Fall bildet die Schwimmeinrichtung mit ihrer der Dichtlippe des zweiten Dichtelementeszugewandten Seite den einen Teil der Dichtung und die Dichtlippe selbst den anderen Teil der zweiten Dichtung, so daß somit eine einfache Lösung für das Dichtmittel ohne weitere bewegliche Teile geschaffen werden kann, die, falls vorhanden, nur zu Funktionsstörungen neigen würden.

Die zweite Dichtlippe liegt vorzugsweise im Zustand der Ausbildung als Teilinnenraum nach Art des Bypasses dichtend auf der Schwimmeinrichtung auf, wobei, wie vorangehend erläutert, in diesem Falle die Schwimmeinrichtung selbst das nötige Dichtpendant zur Dichtlippe darstellt, d.h. die Dichtlippe und die Schwimmeinrichtung selbst bilden die Dichtung, wodurch auf andere bewegliche Unterbrechungs- oder Dichtungsteile bisheriger Befeuchtungsvorrichtungskonstruktionen vollständig verzichtet werden kann, so daß auch Stößevon außen auf die Befeuchtungsvorrichtung weitgehend keine Wirkung zeigen, denn der Schwimmkörper ist im Bereich der an ihn angrenzenden Dichtlippe so beschaffen, daß auf die Vorrichtung von außen sogar lateral einwirkende Schockbelastungen faktisch keine Wirkung zeigen, d.h. die Dichtwirkung nicht beeinträchtigen.

Vorteilhafterweise besteht wenigstens die Dichtlippe aus einem elastomeren Werkstoff und ebenfalls bestehtvorzugsweise das kragenartige Element der Schwimmeinrichtung ebenfalls aus einem elastomeren Werkstoff. Die Elastizität des Werkstoffs sowohl der Dichtlippe als auch des kragenartigen Elements kann, je nach Konstruktion und Einsatz der Befeuchtungsvorrichtung, unterschiedlich und angepaßt an die konstruktiven und betriebsmäßigen Anforderungen der Befeuchtungsvorrichtung ausgebildet werden.

Wie oben erwähnt, kann die Schwimmeinrichtung aus metallischem aber auch aus unmetallischem Werkstoff bestehen, es ist abervorteilhaft, sie aus einem Kunststoffwerkstoff auszubilden, um so die Schwimmeinrichtung als einheitliches Spritzgußteil aus Kunststoff herstellen zu können, was außer dem geringen Gewicht, den geringen Herstellungskosten und der zu erwartenden Betriebssicherheit auch noch den Vorteil hat, daß schon beim Herstellungsvorgang der Schwimmeinrichtung Keimfreiheit gewährleistet sein kann.

Auch das Behältergehäuse selbst kann vorzugsweise aus Kunststoffwerkstoff hergestellt sein, vorzugsweise aus spritzfähigem Kunststoff, wobei vorzugsweise der Einlaß für die Zufuhr des Wassers in den Behälter und das Behälteroberteil einstückig miteinander ausgebildet sein können, d.h. der Behälter bzw. das Behältergehäuse kann faktisch in einem einzigen Arbeitsschritt aus Kunststoff, insbesondere spritzfähigem Kunststoff, hergestellt werden, wodurch ebenfalls schon durch einen derart gewählten Herstellungsvorgang auch hier Keimfreiheit gewährleistet sein kann und aufwendige Prozeduren, um die Befeuchtungsvorrichtung keimfrei zu machen, weitgehend entfallen können.

Nicht nur wenigstens das Behälteroberteil und/oder nicht nur wenigstens der Einlaß für die Zufuhr des Wassers können vorzugsweise aus Kunststoffwerkstoff, insbesondere spritzfähigem Kunststoffwerkstoff, ausgebildet werden, vielmehr kann auch der Boden des Behälters, der mit dem Behältergehäuse nach der Ausrüstung der Befeuchtungsvorrichtung mit dem Mittel, d.h. den Ventilen sowie des Schwimmelements konfektioniertwerden muß, bevorermiteinem hinterheraus Metall bestehenden Behälterboden wasserdicht verschlossen wird, vielmehr ist es außerordentlich vorteilhaft, den Behälterboden ebenfalls aus Kunststoff, insbesondere spritzfähigem Kunststoff, auszubilden, um so einerseits auch eine Keimfreiheit schon im Zuge der Herstellung des Behälters bzw. seiner Einzelteile zu gewährleisten und andererseits auch die Befeuchtungsvorrichtung noch leichterauszubilden und diese auch nach i.d.R. einmaligem Gebrauch leichter und damit kostengünstiger entsorgen zu können, dafaktisch keine metallischen Teile in der erfindungsgemäßen Befeuchtungsvorrichtung vorhanden sind und den sog. Recyclevorgang fürdie einzelnen Komponenten der Befeuchtungsvorrichtung auf die einfachste Weise ausführbar zu machen.

Die im Stand der Technik bei den Befeuchtungsvorrichtungen bekannten Behälterböden sind regelmäßig aus Metall bzw. Metallegierungen ausgebildet, wobei diese metallischen Behälterböden in einem verhältnismäßig aufwendigen Verbindungsvorgang mit dem regelmäßig aus Kunststoff bestehenden Behälter- bzw. Behälteroberteil verbunden werden.

Vorzugsweise wird deshalb gem. der Erfindung der Behälterboden aus Kunststoffwerkstoff ausgebildet, was eine insbesondere dann sehr einfache Verbindung mit dem Behälteroberteil erlaubt, wenn Behälteroberteil und Behälterboden aus dem gleichen Kunststoffwerkstoff bestehen.

Regelmäßig muß das im Behälteroberteil aufgenommene Wasser zur bestimmungsgemäßen Ausbildung von Wasserdampf für die bestimmungsgemäß zu bewirkende Befeuchtung der Lufttemperiertwerden, d.h. auf die Temperatur gebracht und dort gehalten werden, die eine physiologisch für den bestimmungsgemäßen Zweck geeignete Temperatur bzw. einen geeigneten Temperaturbereich darstellt. Zu diesem Zweck werden die Befeuchtungsvorrichtungen im Bodenbereich auf eine Temperiereinrichtung gestellt bzw. geschoben, wobei dabei sichergestellt ist, daß ein guter Wärmeübergang zwischen der Temperiereinrichtung und dem Behälterboden sichergestellt ist.

Es hat sich herausgestellt, daß sich dazu am besten, d.h. für eine schnelle Temperierung und einer Vermeidung von Wärmeverlusten beim Temperiervorgang außerordentlich gut ein Behälterboden eignet, der aus einer aus einem Kunststoffwerkstoff bestehenden Folie ausgebildet ist. Vorzugsweise ist der Kunststoffwerkstoff des Behälterboden Polyester, wobei vorzugsweise die Dicke des Behälterbodens im Bereich von 0,05 bis 0,5 mm liegt.

Die Erfindung wird nun unter Bezugnahme auf die nachfolgenden schematischen Zeichnungen anhand eines Ausführungsbeispieles beschrieben. Darin zeigen:
- Fig. 1: in schematischem Halbschnitt die Befeuchtungsvorrichtung gem. der Erfindung, aus der die wesentlichen Elemente der Vorrichtung ersichtlich sind, in gegenüber einer realisierten Normalgröße geringfügig verkleinerten Darstellung,
- Fig. 2-4: eine Darstellung gem. Fig. 1, bei der die jeweiligen Behälterinnenräume mit unterschiedlichen Wassermengen gefüllt sind, ausgehend von Fig. 2 mit geringem Wasserinhalt über Fig. 3 mit erhöhtem Wasserinhalt und Fig. 4 mit einem maximal möglichen Wasserinhalt, bei der die Wasserzufuhr in den Teilraum des Behälterinnenraums unterbrochen ist,
- Fig. 5: in perspektivischer Darstellung die Befeuchtungsvorrichtung mit einem abnehmbaren Schutzring, mit dem auch der Lufteinlaß und der Luftauslaß im Lagerzustand der Befeuchtungsvorrichtung verschlossen sind und
- Fig. 6: in perspektivischerDarstellung das in derfreien Öffnung des Rohres aufgenommene Ventil in stark vergrößerter Form im Zustand des entspannten tellerförmigen Abschnitts des Ventils.

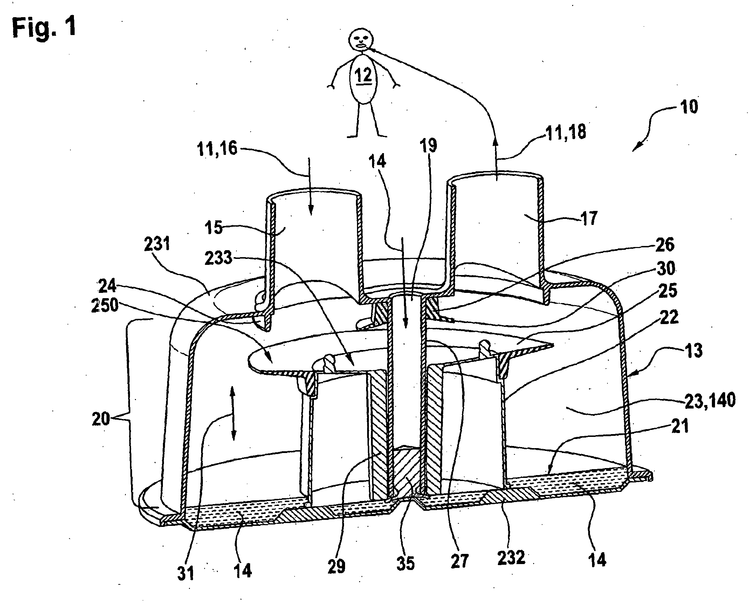

Es wird zunächst Bezug genommen auf die Darstellung gem. Fig. 1, anhand der der grundsätzliche Aufbau der Befeuchtungsvorrichtung 10 beschrieben wird.

Die Befeuchtungsvorrichtung 10 umfaßt einen im wesentlichen geschlossenen Behälter 13, in den das die Atemluft 11 befeuchtende Wasser 14 aufgenommen wird. Der Behälter 13 weist eine erste Öffnung 15 auf, über die Luft 16, die von einer abgesetzt angeordneten Luftquelle über einen Schlauch oder dgl. herangeführt wird, die unbefeuchtet ist. Darüber hinaus weist die Befeuchtungsvorrichtung 10 eine zweite Öffnung 17 auf, aus der die im Behälterinnenraum 23 befeuchtete Luft 18 dem Menschen 12 oder dem Tier, das zu beatmen ist, zugeführt wird. Schließlich weist die Befeuchtungsvorrichtung einen Einlaß 19 für die Zufuhr des Wassers 14 in den Behälterinnenraum 23 auf.

Die erste Öffnung 15für die Zufuhrbefeuchteter Luft 16, die zweite Öffnung 17 für den Auslaß für die befeuchtete Luft 18 und der Einlaß 19 für die Zufuhr des Wassers 14 sind im oberen Teil des Behälters 13, hier Behälteroberteil 231 genannt, angeordnet. Der Einlaß und der Auslaß für die Luft 16, 18 sind hier in Form von vom Behälteroberteil 231, bezogen auf die Darstellung von Fig. 1, nach oben wegstehenden Anschlußelementen ausgebildet, und zwar in Form kleiner Rohrstutzen, so daß Luftschläuche zur Befeuchtungsvorrichtung 10 und Luftschläuche von der Befeuchtungsvorrichtung 10 weg zu einem Menschen 12 oder einem Tier mit der Befeuchtungsvorrichtung 10 leicht verbunden werden können.

Der Behälter 13 weist zudem einen Behälterboden 232 auf, wobei das Behälteroberteil 231 und der Behälterboden 232 im wesentlichen den Behälter 13 bilden. Der Behälter 13 weist bei der in den Fig. dargestellten Ausgestaltungen lediglich drei Öffnungen auf, nämlich die erste Öffnung 25, die zweite Öffnung 17 und eine Öffnung, die den Einlaß 19 für die Zufuhr des Wassers 14 bildet. Es ist aber auch möglich, hier nicht dargestellt, im oberen Bereich des Behälterinnenraumes 23 weitere Öffnungen vorzusehen, in denen bspw. Wasserproben entnommen werden können oder aber Temperaturfühler für das Wasser 14 und die befeuchtete Luft 18 an- bzw. eingebracht werden können. Der Behälter 13 bzw. das Behälteroberteil 231 weist im Querschnitt die Form einer umgekehrten flachen Tasse auf, es sei aber darauf hingewiesen, daß die Form des Behälters 13 bzw. des Behälteroberteils 231 an sich in Abhängigkeit der für den bestimmungsgemäßen Einsatzausgestaltung der Befeuchtungsvorrichtung 10 beliebig geeignet ausgestaltet sein kann, z.B. auch rechteckförmig im Querschnitt u.dgl.

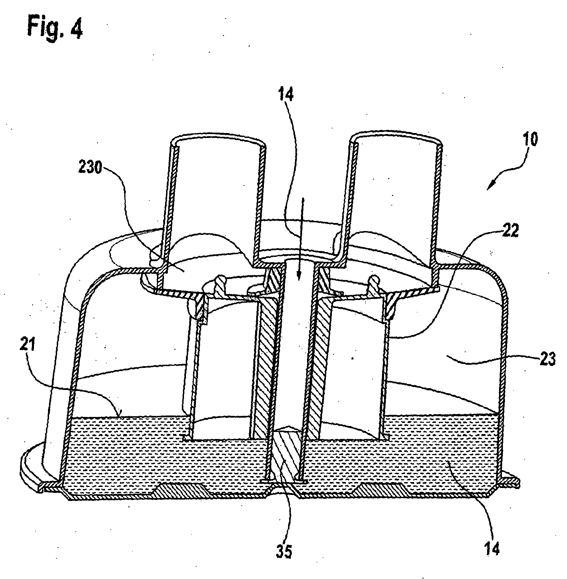

Im Behälteroberteil 231, den Einlaß 19 für Wasser 14, die erste Öffnung 15 für die unbefeuchtete Luft 16 und die zweite Öffnung für den Auslaß der befeuchteten Luft 18 innen ringförmig einfassend, ist ein erstes, im Querschnittvom Behälteroberteil 231 in den Behälterinnenraum 23 hineinstehend stegartig ausgebildetes Dichtelement 24 vorgesehen, was im Zustand der Befeuchtungsvorrichtung 10 gem. Fig. 4 an ein kragenartiges Element 25 anstößt, das am oberen Ende der Schwimmeinrichtung 22 angeordnet ist, bezogen auf die Darstellungen der Befeuchtungsvorrichtung in den Figuren, was aber im einzelnen noch weiter unten beschrieben wird.

Im Behälterinnenraum 23 ist bei der in den Figuren dargestellten Ausgestaltung der Befeuchtungsvorrichtung die hier im wesentlichen rotationssymmetrisch im Längsquerschnitt ausgebildete Schwimmeinrichtung 22 angeordnet, die als Teil eines Mittels 20 vorgesehen ist, das in Abhängigkeit des Pegelstandes 21 des Wassers im Behälterinnenraum 23 vertikal in Richtung des Pfeiles 31 hin-und herbewegbar ist. Mit dem Mittel 20 wirkt auch das erste Dichtelement 24 zusammen, d.h. genauer mit der Schwimmeinrichtung 22. Die Schwimmeinrichtung 22 ist derart beschaffen, daß sie im Wasser 14 schwimmt, das über den Einlaß 19 durch ein im wesentlichen zentral im Behälteroberteil 231 ausgebildetes, vertikal verlaufendes Rohr 27 am vertikal unteren Ende des Rohres austritt und sich oberhalb des Behälterbodens 232 im Innenraum 23 verteilt. Das Rohr 27 verläuft in einem im wesentlichen zentralen Durchtritt 29 der Schwimmeinrichtung 22 und wird dort im wesentlichen durch das Rohr 27 zentriert geführt. Die Führung ist so beschaffen, daß der Reibungswiderstand gegenüber dem Rohr 27 sehr klein ist. Längs des Rohres 27 kann sich somit in Abhängigkeit des Pegels 21 des Wassers 14 im Behälterinnenraum 23 die Schwimmeinrichtung 22 in Richtung des besagten Pfeiles 31 hin- und herbewegen, und zwar in Abhängigkeit, wie gesagt, des Füllstandes des Behälterinnenraumes 23 mit Wasser 14. Die Schwimmeinrichtung 22 kann als im wesentlichen geschlossener, schwimmförmiger Körper ausgebildet sein.

Das Mittel 20 bzw. die Schwimmeinrichtung 22 weist ein umlaufendes, kragenartiges Element 25 auf Es ist an der Schwimmeinrichtung 22, bezogen auf die in den Figuren oberen Bereiche, befestigt und steht im wesentlichen mit seiner radial ausgerichteten Dichtfläche 250 derart von einer gedachten, durch das Rohr 27 hindurchgehenden zentralen Achse nach außen, daß sie bis unter das erste Dichtelement 24 ragt.

Das Mittel 20 umfaßt darüber hinaus auch noch ein Dichtelement 26 aus elastischem polymeren Werkstoff, das im Behälterinnenraum im Behälteroberteil 231 um den Einlaß 19 für Wasser 14 herum, hier als im wesentlichen rotationssymmetrisches Teil, ausgebildet ist. Auch das kragenartige Element 25 besteht aus elastischem, polymerem Werkstoff. Das zweite Dichtelement 26 kann einstückig mit dem Behälteroberteil 231 ausgebildet sein, es kann aber auch als gesondertes Teil hergestellt werden und im Zuge der Montage der Befeuchtungsvorrichtung 10 fest und dichtend mit dem Behälteroberteil 231 verbunden werden. Das zweite Dichtelement 26 steht einer oberen, bezogen auf die Darstellung in den Figuren, radial, bezogen auf das Rohr 27, wegstehend ausgebildeten Anlageebene233 gegenüber, die mit einer umlaufenden Dichtlippe 30 des zweiten Dichtelements 26 zusammenwirkt und die Zufuhrvon Wasser 20 in den Teilinnenraum 230 des Behälters 13 mittelbar unterbricht, wenn das erste Dichtelement 24 an der radialen Dichtfläche 250 und das zweite Dichtelement 26 ebenfalls an der radialen Dichtfläche 250 anliegen.

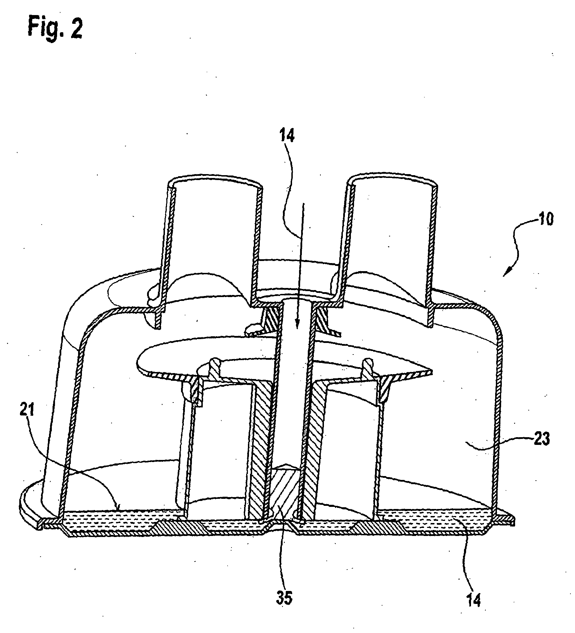

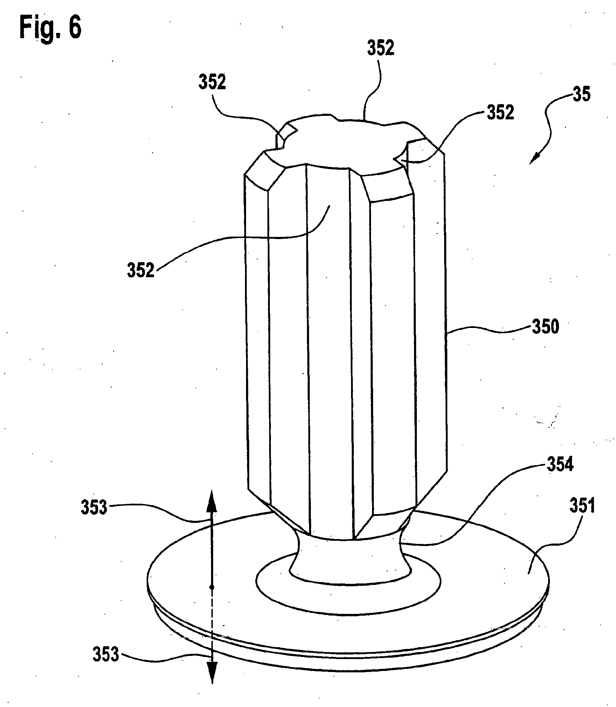

Die in der Fig. verwendeten Bezugsziffern gelten gleichermaßen für die Fig. 2, 3, 4 und 5 sowie 6. Einige der Bezugsziffern sind insbesondere in den Fig. 2, 3 und 4 aus Übersichtlichkeitsgründen weggelassen worden.

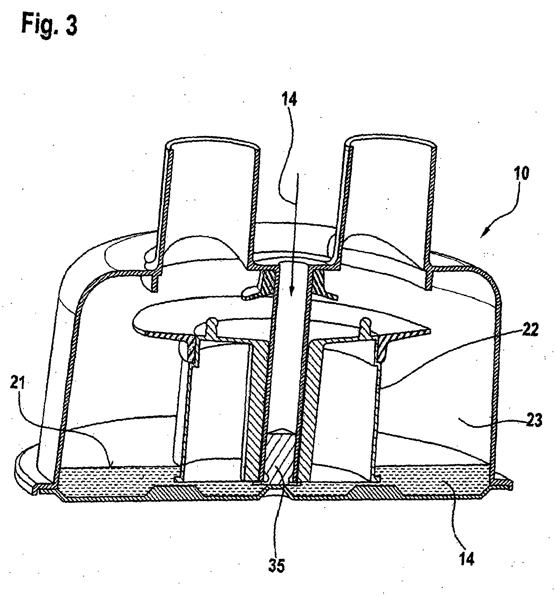

Es wird nun Bezug genommen auf die Darstellung der Fig. 2 bis 4, die einen Grundmodus bzw. Öffnungsmodus für Wasser 14, einen mittleren Wasserfüllgradmodus, Fig. 3, zeigen und einen Füllgradfür Wasser 14, Fig. 4, zeigen, bei dem der Teilinnenraum 230 vom übrigen Behälterinnenraum 23 abgeschlossen ist. Fig. 4 zeigt quasi einen Sicherheitszustand der Vorrichtung 10 für den Menschen oder das Tier, worauf noch weiter unten im einzelnen eingegangen wird. Dabei sind die erste Öffnung 15 für den Einlaß derzu befeuchtenden Luft und die zweite Öffnung 17 für den Auslaß der befeuchteten Luft 18 jeweils mit einer nicht dargestellten Luftquelle 11 bzw. mit dem zu beatmenden Menschen 12 verbunden und es ist der Einlaß 19 mit einer Quelle für Wasser 14 zum Befeuchten der Luft 11, 16, 18 im Behälterinnenraum 230 verbunden.

Bei der Inbetriebnahme der Vorrichtung 10 wird zunächst Wasser 14 zugeführt, so daß sich im Behälterinnenraum 23 ein bestimmter Pegelstand 21 des Wassers 14 einstellt.

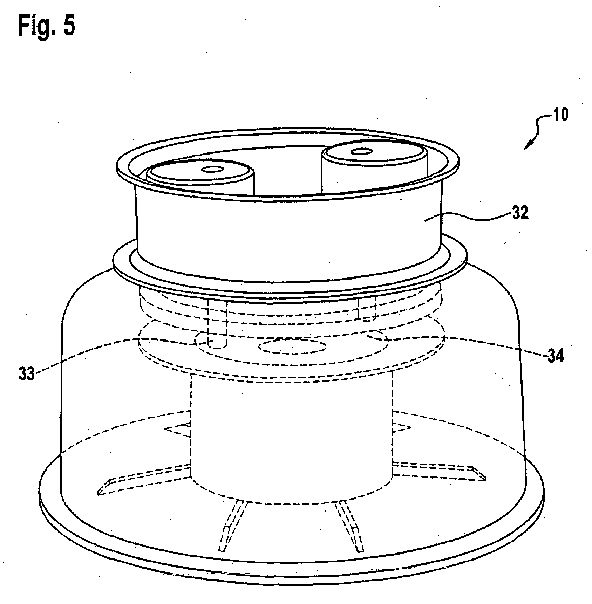

Von einer hier nicht dargestellten Wasserquelle wird Wasser 14 in den Einlaß 19 der Vorrichtung 10 gegeben. Die Wasserquelle steht unter einem geringfügig höheren Umgebungsdruck als der Druck im Behälterinnenraum 23 groß ist. Das Wasser 14 trifft auf ein am freien Ende 270 des Rohres 27 angeordnetes Ventil 35. Das Ventil 35 ist in bezug auf seinen wesentlichen Aufbau in der Fig. 5 dargestellt. Das Ventil 35 weist einen im wesentlichen zylinderförmigen Abschnitt 350 auf und einen im wesentlichen tellerförmigen Abschnitt 351. Mit seinem zylinderförmigen Abschnitt 350 sitzt das Ventil 25 im freien Ende 270 des Rohres. Mit demtellerförmigen Abschnitt351 wird die Öffnung271 des freien Endes 270 des Rohres 27 verschlossen. Wenigstens der tellerförmige Abschnitt 371 des Ventils 35 besteht aus einem hochelastischen, elastomeren Werkstoff, bspw. Silikongummi.

Der zylinderförmige Abschnitt 350 des Ventils 35 weist eine Mehrzahl axial verlaufender Rinnen 352 auf, durch die Wasser in Richtung der Öffnung 271 des freien Endes 270 des Rohres 27 gelangen kann.

Da der Druck im Behälterinnenraum 23, d.h. im zunächst wasserfreien Zustand, zunächst so eingestellt ist, daß der Außendruck, mit dem das Wasser 14 über das Ventil 35 und dem demzufolge geringfügigen Verschwenken des tellerförmigen Abschnitts 351 in Richtung des Pfeiles 353 nach unten, bezogen auf die Fig. 5, strömt, größer ist, wird so lange Wasser 14 über das Ventil 35 in den Behälterinnenraum 23 geführt, bis der Pegelstand 21 des über das Rohr 27 zugeführten Wassers erreicht ist, bei dem der Behälterboden 232 im Behälterinnenraum 23 im wesentlichen mit Wasser 14 bedeckt ist. Der Pegelstand 21 wird durch die mechanische Konstruktion der Vorrichtung 10 sowie den vorherrschenden bzw. gewünschten Druckverhältnissen des zugeführten Wassers 14 sowie der zugeführten Atemluft 11 bzw. der abgeführten befeuchteten Luft 18 bestimmt.

Ist der Behälterboden 232 mit Wasser 14 bis zum Erreichen eines bestimmten Pegelstandes 21 bedeckt, hat sich infolgedessen der Druck im Behälterinnenraum 23 erhöht, so daß der tellerförmige Abschnitt 351 sich, bezogen auf die Darstellung von Fig. 5, in Richtung des Pfeiles 353 nach oben bewegt, so daß der tellerförmige Abschnitt 351 die Öffnung 271 des freien Endes 270 des Rohres 27 verschließt und kein weiteres Wasser 27 in den Behälterinnenraum 23 gelangen kann. Das ist der normale Betriebszustand der Vorrichtung 10 gem. Fig. 2. Es reicht für den Befeuchtungsvorgang der Atemluft 11 bei ihrem Durchtritt durch die Vorrichtung 10, wenn lediglich der Behälterboden 232 mit Wasser 14, maximal einige Millimeter hoch, bedeckt ist. Dadurch wird auch der Energiebedarf für die Erwärmung des Wassers 14 zur Erzeugung des Wasserdampfes 140 im Behälterinnenraum 23 begrenzt.

Aufgrund fehlerhafter Zufuhr von Wasser 10 zur Vorrichtung, bei Falschbedienung der Vorrichtung 10 aber auch bei einem Defekt des Ventils 35, das ein wichtiges Funktionselement der Vorrichtung 10 ist, kann der Zustand eintreten, daß ungewollt Wasser 14 in den Behälterinnenraum 23 über den vorbeschriebenen Weg eintritt.

Bei zulaufendem Wasser 14 erhöht sich im Behälterinnenraum 23 der Pegelstand 21 des Wassers 14, so daß sich die Schwimmeinrichtung 22 als Teil des Unterbrechungsmittels 20 in Richtung des Pfeiles 31 nach oben bewegt, Fig. 3. Wenn weiter Wasser 14 zugeführt wird, steigt der Pegel 21 des Wassers 14 im Behälterinnenraum 23 und die Schwimmeinrichtung 22 stößt mit ihrem kragenartigen Element 25 bzw. der Dichtfläche 250 des kragenartigen Elementes an das erste Dichtelement 24. Die Schwimmeinrichtung 22 kann, selbst wenn Wasserweiterhin zugeführt wird, nichtweiterin Richtung des Pfeiles 31 nach oben ansteigen, denn die Bewegung wird durch die Anlage des ersten Dichtelementes 24 am kragenartigen Element 25 der Schwimmeinrichtung 22, bezogen auf die Darstellung in den Figuren, in vertikaler Richtung begrenzt. Im an das erste Dichtelement 24 angestoßenen Zustand des kragenartigen Elements 25 wird der Einlaß der Luft 16 in den Behälter 13 und der Auslaß der Luft 16 aus dem Behälter 13 über einen verbleibenden Teilinnenraum 230 im wesentlichen nach Art eines Bypass kurzgeschlossen, so daß zwar die zur Beatmung notwendige Atemluft 18 aus derzweiten Öffnung 17 herausgeführt und dem Menschen 12 bzw. dem Tier zugeführt werden kann. In diesem Falle wird die Atemluft 11 nicht befeuchtet, da der Weg über den Bypass keinen Kontakt mit dem Wasserdampf 140 im Behälterinnenraum 23 erlaubt. Durch die im wesentlichen gleichzeitige Anlage des zweiten Dichtelementes 26 an dem kragenartigen Element 25 kann in diesen verbleibenden Teilinnenraum 230 kein Wasser 14 eindringen, da auch das zweite Dichtelement 26 in dichtenden Eingriff mit einer entsprechend ausgebildeten Oberfläche, s.o., der Schwimmmeinrichtung 22 kommt und somit ein Eindringen des Wassers 14 über den Durchtritt 29 zwischen Rohr 27 und der Schwimmeinrichtung 22 in den im Zustand der Fig. 4 gebildeten Teilinnenraum 230 verhindert.

Gem. der Erfindung wird somit bei ungestörtem Betriebsablauf erreicht, daß in Abhängigkeit des Pegelstandes 21 des Wassers 14 in den Behälter 13 die Zufuhr von Wasser 14 in den Behälter 13 beim Erreichen eines vorbestimmten Pegelstandes 21 unterbrochen wird und bei Unterschreiten des Pegelstandes 21 die Zufuhr von Wasser 14 in den Behälter 13 erlaubt wird. Bei Störungen im Betriebsablauf der Vorrichtung 10, bei denen Wasser 14 über den gewünschten Pegelstand 21 hinaus in den Behälterinnenraum 23 eingeführt wird, wird der Zugang des Wassers 14 in den Teilinnenraum 230 verhindert, s.o., so daß die Atemluft 11 für Mensch 12 und Tier nicht mit Wasser 14 beaufschlagt wird. Es sind somit erfindungsgemäß keine komplizierten Ventilteile, die störanfällig sind, vonnöten, um zu gewährleisten, daß niemals Wasser in den Bereich des Auslasses für die befeuchtete Luft 18 gelangt, wobei man hiervon einem quasi selbstregelnden System sprechen kann.

Bei der Ausführungsform gem. Fig. 5 ist die erfindungsgemäße Befeuchtungsvorrichtung 10 mit einer abnehmbaren Transportsicherung bzw. Transportkappe 32 versehen, die im Zustand der Lagerung und des Transportes aber auch bei momentanem Nichtgebrauch den Bereich der Rohrstutzen der ersten Öffnung 15, Lufteinlaß, den Bereich der zweiten Öffnung 17, Luftauslaß, und den Bereich des Einlasses 19 für das Wasser 14, letzteres in Fig. 5 nicht direkt sichtbar, abdeckt. Zusammen mit der Transportkappe 23 können durch die erste Öffnung 15 und die zweite Öffnung 17 hindurchragende stangenartige Vorsprünge ausgebildet sein, die in diesem Zustand, quasi auch als Transporthalterung 33, 34, die Schwimmeinrichtung 22 am Behälterboden 232 angedrückt halten. Wenn die Transportkappe 32 durch geringfügigen Zug in Richtung des Pfeiles 31 nach oben abgezogen wird, sind die erste Öffnung 15 und die zweite Öffnung 17 sowie der Lufteinlaß 19 für Wasser 14 freigelegt und gleichzeitig werden die mit derTransportkappe 32 verbundenen beiden stangenförmigen Transporthalterungen 33, 34 entfernt und die Befeuchtungsvorrichtung 10 steht dann zu ihrem bestimmungsgemä-βen Gebrauch zur Verfügung.

### Bezugszeichenliste

- 10: Vorrichtung (Befeuchtungsvorrichtung)
- 11: Atemluft
- 12: Mensch
- 13: Behälter
- 14: Wasser
- 140: Wasserdampf
- 15: erste Öffnung (Lufteinlaß)
- 16: Luft (unbefeuchtet)
- 17: zweite Öffnung (Luftauslaß)
- 18: Luft (befeuchtet)
- 19: Einlaß (Wasser)
- 20: Mittel
- 21: Pegelstand
- 22: Schwimmeinrichtung
- 23: Behälterinnenraum
- 230: Teilinnenraum
- 231: Behälteroberteil
- 232: Behälterboden
- 233: Anlageebene
- 24: erstes Dichtelement (am Behälteroberteil)
- 25: kragenartiges Element
- 250: radiale Dichttläche
- 26: zweites Dichtelement (um den Wassereinlaß herum)
- 27: zentrales Rohr (Wassereinlaß)
- 270: freies Ende des Rohres
- 271: Öffnung des Rohres
- 28: Bodenbereich
- 29: Durchtritt
- 30: Dichtlippe
- 31: Pfeil
- 32: Transportkappe
- 33: Transporthalterung
- 34: Transporthalterung
- 35: Ventil
- 350: zylindrischer Abschnitt
- 351: tellerförmiger Abschnitt
- 352: Rinne
- 353: Pfeil
- 354: Hinterschneidung

## Patentansprüche

1. Vorrichtung (10) zur Befeuchtung von Atemluft (11) für die künstliche Beatmung von Humeniden, insbesondere des Menschen (12), umfassend wenigstens einen im wesentlichen geschlossenen Behälter (13) zur Aufnahme von Wasser (14), wobei der Behälter (13) eine erste Öffnung (15) zum Einlass der unbefeuchteten Luft (16) aufweist sowie eine zweite Öffnung (17) zum Auslass der befeuchteten Luft (18) aufweist und einen Einlass (19) für die Zufuhr des Wassers (14) in den Behälter (13), **dadurch gekennzeichnet, dass** der Behälterboden (232) aus Kunststoffwerkstoff ausgebildet ist, wobei der Behälterboden so ausgeführt ist, dass die Vorrichtung zur Temperierung des Wassers mit dem Behälterboden (232) auf einer Temperiereinrichtung gestellt werden kann.

2. Befeuchtungsvorrichtung nach Anspruch 1, **dadurch gekennzeichnet, dass** der Kunststoffwerkstoff des Behälterbodens (232) spritzfähig ist.

3. Befeuchtungsvorrichtung nach Anspruch 1, **dadurch gekennzeichnet, dass** der Behälterboden (232) und ein Behälteroberteil (231) aus dem gleichen Kunststoffwerkstoff bestehen.

4. Befeuchtungsvorrichtung nach Anspruch 1, **dadurch gekennzeichnet, dass** der Kunststoffwerkstoff nach Art einer Folie ausgebildet ist.

5. Befeuchtungsvorrichtung nach Anspruch 1, **dadurch gekennzeichnet, dass** der Kunststoffwerkstoff Polyester ist.

6. Befeuchtungsvorrichtung nach Anspruch 1, **dadurch gekennzeichnet, dass** die Dicke des Behälterbodens im Bereich von 0,05 bis 0,5 mm liegt.

7. Befeuchtungsvorrichtung nach Anspruch 1, **dadurch gekennzeichnet, dass** der Behälterboden (232) Aussteifungen in Form von Verdickungen des Behälterbodens (232) beinhaltet.

8. Befeuchtungsvorrichtung nach Anspruch 1, **dadurch gekennzeichnet, dass** der Behälterboden (232) Aussteifungen in Form von sich radial erstreckenden Latten beinhaltet.

9. Befeuchtungsvorrichtung nach Anspruch 1, **dadurch gekennzeichnet, dass** der Einlass (19) im Behälteroberteil (231) ausgebildet ist, an den sich ein den Behälterinnenraum (23) im wesentlichen durchquerendes Rohr (27) anschließt, wobei am freien Ende (270) des Rohres (27) ein Ventil (35) angeordnet ist, das den Pegelstand (21) des über das Rohr (27) zugeführten Wassers (14) begrenzt.

10. Befeuchtungsvorrichtung nach Anspruch 9, **dadurch gekennzeichnet, dass** das Ventil (35) einen im wesentlichen zylinderförmigen Abschnitt (350) aufweist, mit dem es im freien Ende (270) des Rohres (27) aufgenommen wird, und einen im wesentlichen tellerförmigen Abschnitt (351), mit dem es die Öffnung (271) des freien Endes (270) des Rohres (27) verschließt.

11. Befeuchtungsvorrichtung nach Anspruch 9, **dadurch gekennzeichnet, dass** der Behälterboden (232) eine zentrale Erhebung aufweist auf der das Ventil (35) anliegt.

12. Befeuchtungsvorrichtung nach Anspruch 11, **dadurch gekennzeichnet, dass** der äußere Durchmesser des tellerförmigen Abschnitts (351) grösser ist als der der Erhebung des Behälterbodens (232).

13. Befeuchtungsvorrichtung nach Anspruch 9, **dadurch gekennzeichnet, dass** das im wesentlichen zentrale Rohr (27) sich im wesentlichen bis in den Bodenbereich (28) des Behälters (13) erstreckt.

14. Befeuchtungsvorrichtung nach Anspruch 1, **dadurch gekennzeichnet, dass** der Einlass (19) für die Zufuhr des Wassers (14) in den Behälter (13) und das Behälteroberteil (231) einstückig miteinander ausgebildet sind.

15. Befeuchtungsvorrichtung nach Anspruch 1, **dadurch gekennzeichnet, dass** keine Metallischen Teile in der Befeuchtungsvorrichtung vorhanden sind.

## Claims

1. An apparatus (10) for humidifying the respiratory air (11) for artificial respiration of humanoids, in particular humans (12), comprising at least one essentially closed container (13) to accommodate water (14), wherein the container (13) has a first opening (15) for an inlet for the unhumidified air (16) as well as a second opening (17) for the outlet of the humidified air (18) and has an inlet (19) for supplying water (14) into the container (13), **characterized in that** the container bottom (232) is made of a plastic material, wherein the container bottom is designed so that the apparatus for regulating the temperature of the water and having the container bottom (232) can be placed on a temperature-regulating apparatus.

2. The humidifying apparatus according to Claim 1, **characterized in that** the plastic material of the container bottom (232) is injectable.

3. The humidifying apparatus according to Claim 1, **characterized in that** the container bottom (232) and a container top part (231) are made of the same plastic material.

4. The humidifying apparatus according to Claim 1, **characterized in that** the plastic material is designed in the manner of a film.

5. The humidifying apparatus according to Claim 1, **characterized in that** the plastic material is polyester.

6. The humidifying apparatus according to Claim 1, **characterized in that** the thickness of the container bottom is in the range of 0.05 to 0.5 mm.

7. The humidifying apparatus according to Claim 1, **characterized in that** the container bottom (232) includes reinforcements in the form of thickened areas of the container bottom (232).

8. The humidifying apparatus according to Claim 1, **characterized in that** the container bottom (232) contains reinforcements in the form of strips extending radially.

9. The humidifying apparatus according to Claim 1, **characterized in that** the inlet (19) is formed in the container top part (231), to which is connected a pipe (27), which essentially crosses through the container interior (23), wherein a valve (35) which limits the level (21) of the water (14) supplied through the pipe (27) is arranged on the free end (270) of the pipe (27).

10. The humidifying apparatus according to Claim 9, **characterized in that** the valve (35) has an essentially cylindrical section (350) with which it is accommodated in the free end (270) of the pipe (27) and has an essentially disk-shaped section (351) with which it closes the opening (271) of the free end (270) of the pipe (27).

11. The humidifying apparatus according to Claim 9, **characterized in that** the container bottom (232) has a central elevation with which the valve (35) is in contact.

12. The humidifying apparatus according to Claim 11, **characterized in that** the outside diameter of the disk-shaped section (351) is larger than the outside diameter of the elevation of the container bottom (232).

13. The humidifying apparatus according to Claim 9, **characterized in that** the pipe (27), which is basically central, extends essentially to the bottom area (28) of the container (13).

14. The humidifying apparatus according to Claim 1, **characterized in that** the inlet (19) for the supply of water (14) into the container (13) and the container top part (231) are designed in one piece with one another.

15. The humidifying apparatus according to Claim 1, **characterized in that** no metallic parts are present in the humidifying apparatus.

## Revendications

1. Dispositif (10) d'humidification d'air respiratoire (11) pour la respiration artificielle d'humanoïdes, en particulier de l'humain (12), comprenant au moins un récipient (13) essentiellement fermé pour recevoir de l'eau (14), dans lequel le récipient (13) présente une première ouverture (15) pour admettre l'air non humidifié (16) ainsi qu'une seconde ouverture (17) pour évacuer l'air humidifié (18) et une admission (19) pour l'amenée d'eau (14) dans le récipient (13), **caractérisé en ce que** le fond du récipient (232) est en plastique , le fond du récipient étant ainsi formé que le dispositif peut être posé sur un dispositif de mise en température par le fond du récipient (232) pour la mise en température de l'eau.

2. Dispositif d'humidification selon la revendication 1, **caractérisé en ce que** le matériau plastique du fond du récipient (232) est injectable.

3. Dispositif d'humidification selon la revendication 1, **caractérisé en ce que** le fond du récipient (232) et une partie supérieure du récipient (231) sont fabriqués dans le même matériau plastique.

4. Dispositif d'humidification selon la revendication 1, **caractérisé en ce que** le matériau plastique est formé à la manière d'un film.

5. Dispositif d'humidification selon la revendication 1, **caractérisé en ce que** le matériau plastique est du polyester.

6. Dispositif d'humidification selon la revendication 1, **caractérisé en ce que** l'épaisseur du fond du récipient est située dans la plage de 0,05 à 0,5 mm.

7. Dispositif d'humidification selon la revendication 1, **caractérisé en ce que** le fond du récipient (232) comporte des renforts sous forme d'épaississements du fond du récipient (232).

8. Dispositif d'humidification selon la revendication 1, **caractérisé en ce que** le fond du récipient (232) comporte des renforts sous forme de lattes s'étendant radialement.

9. Dispositif d'humidification selon la revendication 1, **caractérisé en ce que** l'admission (19) est formée dans la partie supérieure du récipient (231) sur laquelle se raccorde un tube (27) traversant essentiellement l'intérieur du récipient (23), sachant que sur l'extrémité libre (270) du tube (27) est disposée une vanne (35) qui limite le niveau **(21)** de l'eau (14) amenée via le tube (27).

10. Dispositif d'humidification selon la revendication 9, **caractérisé en ce que** la vanne (35) présente une section (350) essentiellement cylindrique avec laquelle elle est reçue dans l'extrémité libre (270) du tube (27) et une section (351) essentiellement en forme d'assiette avec laquelle elle ferme l'ouverture (271) de l'extrémité libre (270) du tube (27).

11. Dispositif d'humidification selon la revendication 9, **caractérisé en ce que** le fond du récipient (232) présente un bossage central sur lequel repose la vanne (35).

12. Dispositif d'humidification selon la revendication 11, **caractérisé en ce que** le diamètre extérieur de la section **(351)** en forme d'assiette est plus grand que celui du bossage du fond du récipient (232).

13. Dispositif d'humidification selon la revendication 9, **caractérisé en ce que** le tube (27) essentiellement central s'étend essentiellement jusque dans la partie de fond (28) du récipient (13).

14. Dispositif d'humidification selon la revendication 1, **caractérisé en ce que** l'admission (19) pour l'entrée de l'eau (14) dans le récipient (13) et la partie supérieure du récipient (231) sont formées d'un seul tenant l'une avec l'autre.

15. Dispositif d'humidification selon la revendication 1, **caractérisé en ce qu'**aucune pièce métallique n'est présente dans le dispositif d'humidification.
